# FASCICULE DE BREVET EUROPEEN

(11) **EP 0 667 146 B1**
(45) Date de publication et mention de la délivrance du brevet: **07.04.1999**
(21) Numéro de dépôt: 95400067.5
(22) Date de dépôt: 13.01.1995
(51) Int. Cl.: A61K 7/48, A61K 7/025

(54) **Composition cosmétique ou dermopharmaceutique sous forme de pâte souple et procédé de préparation**
Kosmetische oder dermatologische Zusammensetzung in Form von einen weichen Pasta und Herstellungsverfahren
Cosmetic or dermatologic composition in form of a supple paste and preparation process

(30) Priorité: 25.01.1994 FR 9400756
(43) Date de publication de la demande: 16.08.1995
(73) Titulaire: L'OREAL, 75008 Paris (FR)
(72) Inventeur: Le Bras, Véronique, F-75002 Paris (FR); Miguel, Dolorès, F-92340 Bourg-La-Reine (FR); Pradier, François, F-92260 Fontenay-aux-Roses (FR)
(74) Mandataire: Peuscet, Jacques

(56) Documents cités:
- EP-A- 0 605 284
- WO-A-91/16879
- DE-A- 3 243 017
- DE-A- 3 744 352
- FR-A- 2 232 302

## Description

La présente invention concerne une composition sous forme de pâte souple que l'on peut prélever et appliquer pour le maquillage ou le traitement à l'aide d'un applicateur tel qu'un pinceau ou un stylo-mousse, en particulier une composition pour rouge à lèvres ou pour le traitement des lèvres.

Les rouges à lèvres peuvent, de façon connue, se présenter sous deux formes : sous forme de bâton ou sous forme de pâte souple. La présentation sous forme de bâton, bien que largement utilisée, présente un certain nombre d'inconvénients : il est difficile de bien dessiner les contours des lèvres à l'aide d'un bâton et la tenue du bâton à la chaleur est mauvaise, ce qui peut provoquer son ramollissement et le rendre inutilisable.

Les rouges à lèvres sous forme de pâte souple sont prélevés avec un applicateur, ce qui pallie aux inconvénients des rouges à lèvres en bâton. Mais, jusqu'à présent, par les procédés classiques de fabrication, on ne peut introduire qu'une faible quantité de cires dans les compositions cosmétiques sous forme de pâtes souples, car, avec des quantités croissantes de cires, on augmente la viscosité des pâtes, ce qui rend leur prélèvement et leur application difficiles ; et généralement, on obtient, quand on incorpore des quantités de cires élevées, un solide qui n'est utilisable que sous forme de bâton.

Or, les cires jouent un rôle important dans les qualités cosmétiques demandées à une composition cosmétique, notamment à un rouge à lèvres, en particulier les qualités de consistance, onctuosité, tenue du film appliqué et épaisseur dudit film ; les pâtes souples obtenues jusqu'à présent, par exemple selon les brevets US-A-5 085 855 et US-A-4 935 228, ne contiennent généralement pas de cires ou une quantité inférieure à 12 % et elles sont, en conséquence, ressenties par l'utilisateur comme trop huileuses, trop brillantes et manquant de tenue.

La présente invention a pour objet une composition cosmétique ou dermopharmaceutique sous forme de pâte souple, utilisable pour le maquillage ou le traitement de la peau ou des lèvres, contenant au moins une cire dans une phase grasse, caractérisée par le fait qu'elle contient de 12% à 60 % en poids, par rapport au poids total de la composition, d'au moins une cire ayant un point de fusion supérieur à 55°C et qu'elle a une viscosité dynamique à 25°C comprise entre 3 et 30 Pascals-seconde, mesurée avec un viscosimètre rotatif CONTRAVES TV équipé d'un mobile "MS-r4" à la fréquence de 60 Hz.

La composition selon l'invention contient, de préférence, 15 à 40 % en poids de cires ayant un point de fusion supérieur à 55°C.

La composition selon la présente invention ayant une teneur en cires supérieure à 12 % en poids est susceptible, par application en couche, par exemple sur les lèvres, de former un film ayant une bonne tenue. De plus, sa viscosité dynamique étant inférieure à 30 pascals-seconde, elle peut facilement être prélevée et appliquée à l'aide d'un applicateur.

La composition selon la présente invention est donc une pâte souple, dont on peut mesurer la viscosité, par opposition à la structure solide d'un bâton dont on ne peut pas mesurer la viscosité.

Les cires ayant un point de fusion supérieur à 55°C sont, de préférence, des cires ayant un point de fusion compris entre 55 et 110°C et un indice de pénétration à l'aiguille à 25°C comprise entre 3 et 40, telle que mesuré selon la norme française NFT 004 ou la norme américaine ASTM D5. Selon ces normes, l'indice de pénétration à l'aiguille est la mesure de la profondeur, exprimée en dixièmes de millimètre, à laquelle pénètre une aiguille normalisée pesant 2,5 g placée dans un porte-aiguille pesant 47,5 g (soit au total 50 g) placée sur la cire à tester pendant 5 secondes. Selon l'invention, la cire peut être une cire animale, végétale, minérale ou synthétique. Parmi les cires animales, on peut notamment citer les cires d'abeilles. Parmi les cires végétales, on peut citer, entre autres, les cires de Carnauba, de Candellila, d'Ouricurry, les cires de fibres de liège, les cires de canne à sucre et les cires du Japon. Parmi les cires minérales, on peut citer, en particulier, les cires de paraffine, les cires microcristallines, les cires de lignite et les ozokérites. Parmi les cires synthétiques, on peut citer, notamment, les cires de polyéthylène et les cires obtenues par synthèse de Fischer et Tropsch. Toutes ces cires sont bien connues de l'Homme de l'Art. Il faut noter que la cire de lanoline ne correspond pas à la définition des cires donnée ci-dessus.

La phase grasse de la composition peut contenir, de façon connue, outre la (ou les) cire(s) susmentionnée(s), au moins un constituant gras ayant un point de fusion inférieur à 55°C ; ce constituant gras peut être une huile ou un corps gras.

Parmi les huiles susceptibles d'être utilisées en mélange avec la (ou les) cire(s), on peut, en particulier, citer :
- les huiles minérales, telles que l'huile de paraffine, l'huile de vaseline et les huiles minérales ayant un point d'ébullition compris entre 310 et 410°C,
- les huiles d'origine animale telles que le perhydrosqualène, l'huile d'arara,
- les huiles végétales telles que l'huile d'amande douce, l'huile de calophyllum, l'huile de palme, l'huile d'avocat, l'huile de jojoba, l'huile d'olive, l'huile de ricin et les huiles de germes de céréales, telles que l'huile de germes de blé et l'huile de sésame,
- les huiles de silicone, telles que le diméthylpolysiloxane,
- les esters de synthèse, tels que l'huile de Purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le dicaprylate de propylèneglycol, l'adipate de di-isopropyle,
- les alcools organiques, tels que l'alcool oléique, l'alcool linoléique, l'alcool linolénique, l'alcool isostéarylique, l'octyl dodécanol,
- les esters dérivés d'acide lanolique tels que le lanolate d'isopropyle, le lanolate d'isocétyle,
- les esters dérivés d'acide oléique tels que l'oléate d'isopropyle et l'oléate d'isocétyle,
- les acétylglycérides, les octanoates et décanoates d'alcools et de polyalcools, tels que ceux de glycol ou de glycérol, les ricinoléates d'alcools et de polyalcools, tels que celui de cétyle.

Parmi les corps gras susceptibles d'être utilisés en mélange avec la (ou les) cire(s), on peut, en particulier, citer :
- les huiles hydrogénées concrètes à 25°C, telles que l'huile de ricin hydrogénée, l'huile de palme hydrogénée, le suif hydrogéné, l'huile de coco hydrogénée ;
- les esters gras concrets à 25°C, tels que le myristate de propylèneglycol et le myristate de myristyle, l'alcool cétylique ;
- les mono-di- ou triglycérides et les sucroglycérides ;
- les lanolines.

Les constituants gras ci-dessus peuvent représenter 10 à 90 % en poids de la composition.

La composition peut également contenir, de façon connue, au moins un agent de coloration pulvérulent et/ou au moins une charge minérale ou organique pulvérulente.

Comme agents de coloration pulvérulents, on peut mentionner :
- le noir de carbone (CI 77 266), les oxydes de chrome (CI 77 288 et 77 289), les oxydes de fer noir, jaune et rouge (CI 77 499, 77 492, 77 491), les outremers (polysulfures d'aluminosilicates), le pyrophosphate de manganèse, le bleu ferrique (CI 77 510), le dioxide de titane (CI 77 891) et certaines poudres métalliques telles que celles d'argent ou d'aluminium ;
- les agents nacrants, qui sont généralement utilisés en mélange avec des pigments colorés, tels que l'oxychlorure de bismuth, le mica-titane, les cristaux de guanine ;
- certains colorants organiques, qui sont généralement utilisés en mélange avec des pigments colorés, tels que le carmin de cochenille (CI 75 470) et les laques organiques ; ces laques, qui sont couramment employées pour conférer aux lèvres et à la peau un effet de maquillage, sont des sels de calcium, de baryum, d'aluminium ou de zirconium, de colorants acides tels que les colorants halogénoacides, azoïques et anthraquinoniques : parmi ces laques, on peut, en particulier, citer celles connues sous les dénominations suivantes (CI représente la codification dans le "Color Index") : D and C Red 21 (CI 45 380), D and C Orange 5 (CI 45 370), D and C Red 27 (CI 45 410), D and C Orange 10 (CI 45 425), D and C Red 3 (CI 45 430), D and C Red 7 (CI 15 850:1), D and C Red 4 (CI 15 510), D and C Red 33 (CI 17 200), D and C Yellow 5 (CI 19 140), D and C Yellow 6 (CI 15 985), D and C Green 5 (CI 61 570), D and C Yellow 10 (CI 77 002), D and C Green 3 (CI 42 053), D and C Blue 1 (CI 42 090).

Ces agents de coloration peuvent représenter de 0,5 à 20 % en poids par rapport au poids total de la composition.

Les charges pulvérulentes peuvent avantageusement être choisies dans le groupe formé par :
- le talc, qui est un silicate de magnésium hydraté, utilisé sous forme de particules généralement de dimensions inférieures à 40 µm, cette charge possédant des propriétés absorbantes de l'humidité et étant utilisée surtout en raison de son toucher onctueux ;
- les micas, qui sont des aluminosilicates de compositions variées et se présentent sous forme d écailles ayant des dimensions de 2 à 200 µm, de préférence de 5 à 70 µm, et ayant une épaisseur de 0,1 à 5 µm, de préférence de 0,2 à 3 µm, ces micas généralement transparents étant d'origine naturelle (par exemple : muscovite, margarite, roscoelite, lepidolite, biotite) ou synthétique ;
- le kaolin, qui est un silicate d'aluminium hydraté, qui se présente sous la forme de particules de formes isotropes ayant des dimensions généralement inférieures à 30 µm et qui possède de bonnes propriétés d'absorption de corps gras ;
- les oxydes de zinc et de titane, généralement utilisés sous la forme de particules ayant des dimensions ne dépassant pas quelques micromètres, ces oxydes ayant un toucher onctueux, un bon pouvoir couvrant et une opacité importante ;
- le carbonate de calcium précipité qui, sous forme de particules de dimensions inférieures à 10 µm, a un toucher onctueux ;
- le carbonate et l'hydrocarbonate de magnésium, qui possèdent notamment des propriétés de fixation des parfums ;
- la silice, en particulier la silice sphérique et la poudre de silice commercialisée sous la dénomination "CAB-O-SIL TS 530" par la société "CABOT" ;
- le dioxyde de titane sphérique en particulier celui commercialisé sous la dénomination commerciale "SPHERITITAN" ;
- les billes de verre et de céramiques commercialisées par la société "3 M" sous la dénomination commerciale "MACROLITE" ;
- des savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium, ces savons, généralement sous la forme de particules de dimensions inférieures à 10 µm, ayant un toucher onctueux et facilitant l'adhérence de la poudre ;
- les poudres de polymères synthétiques non expansées, tels que le polyéthylène, le polystyrène, les polyesters (par exemple isophtalate ou téréphtalate de polyéthylène), les polyamides (par exemple le nylon ou la poly-β-alanine), les acrylates copolymères (par exemple les microsphères microporeuses vendues par la société "DOW CORNING" sous la dénomination commerciale "POLYTRAP"), les résines de silicone, les acides polyméthacryliques de polystyrène (réticulé par le divinylbenzène), de téflon comme le "FLUON", particules commercialisées par la société "MONTEFLUO", l' "HOSTAFLON Q", particules commercialisées par la société "HOECHST", ces poudres étant constituées de particules ayant des dimensions inférieures à 50 µm, possèdant des propriétés absorbantes et permettant de conférer un aspect velouté ;
- les poudres expansées telles que les microsphères creuses en matériau thermoplastique préparées par des procédés connus, comme ceux décrits dans US-A-3 615 972 et EP-A-0 56219, ces microsphères pouvant être réalisées en tous matériaux thermoplastiques non toxiques et non irritants, par exemple en polymères ou copolymères de dérivés éthyléniques tels que polyéthylène, polystyrène, en copolymère chlorure de vinyle-acrylonitrile, en polyacrylonitrile, en polyamides, en polyesters, en polymères urée-formaldéhyde ou en copolymères de chlorure de vinylidène (tel que le chlorure de vinylidène-acrylonitrile) ; et, notamment, les microsphères commercialisées sous la dénomination commerciale "EXPANCEL" par la société "KEMANORD PLAST" ou sous la dénomination commerciale "MICROPEARL F 80 ED" par la société "MATSUMOTO",
- des poudres de matériaux organiques naturels, tels que les amidons de maïs, de blé ou de riz, réticulés ou non.

Ces charges peuvent représenter jusqu'à 40 % en poids par rapport au poids total de la composition.

La composition peut également contenir, de façon connue, des additifs choisis dans le groupe formé par des adjuvants de formulation et/ou des actifs cosmétiques et/ou dermopharmaceutiques liposolubles.

Comme adjuvant de formulation, on utilise, notamment, au moins un agent antioxydant. Parmi les agents antioxydants, on peut citer les esters propylique, octylique et dodécylique de l'acide gallique, le butylhydroxytoluène et le butylhydroxyanisole. L'agent antioxydant représente généralement, en poids, de 0 à 3 %, de préférence de 0,05 à 0,5 % par rapport au poids total de la composition. L'adjuvant de formulation peut également être un parfum et/ou un conservateur, tel que le parahydroxybenzoate de méthyle ou de propyle.

Les actifs cosmétiques et/ou pharmaceutiques que l'on peut introduire dans la composition sont des composés lipophiles. Parmi ceux-ci, on peut citer, par exemple, les dérivés de vitamines, tels que les esters de tocophérol et les esters de vitamine A, les acides gras essentiels, les sphingocérils et les filtres solaires liposolubles, les antiinflammatoires et les extraits huileux de plantes.

L'actif cosmétique et/ou dermopharmaceutique peut représenter 0,05 à 5 %, de préférence 0,5 à 3 %, en poids par rapport au poids total de la composition.

La composition peut également contenir des polymères liposolubles. Parmi ceux-ci, on peut citer les polyalkylènes (notamment les polyéthylènes et les polybutènes) et les polyacrylates. Parmi les polyalkylènes, on peut citer le polybutène, notamment celui vendu par la société "AMOCO" sous la dénomination commerciale "UDOPOL". Ils peuvent représenter jusqu'à 25 % de la composition.

Jusqu'à présent, pour préparer des compositions cosmétiques ou dermopharmaceutiques sous forme de pâte souple ne contenant pas de cires ou une faible proportion de cire(s), on dispersait le pigment et/ou la charge dans la phase grasse.

Par ailleurs, pour fabriquer des bâtons de rouge à lèvres classiques contenant des cires, on pratiquait de la façon suivante :
- dans une première étape, on chauffait une pâte constituée d'au moins un constituant gras et d'au moins une cire, ladite pâte contenant éventuellement des pigments et/ou des charges et/ou d'autres additifs, à une température supérieure à la température la plus élevée de fusion des cires, dite température finissante,
- dans une seconde étape, on coule la pâte chauffée à la température finissante dans un moule, ce qui équivaut à une première trempe, et le moule est ensuite refroidi, ce qui constitue une seconde trempe.

Dans ce procédé, les deux trempes successives permettent à la (aux) cire(s) de former un réseau cristallin et, par conséquent, d'obtenir un produit solide.

Selon la présente invention, on a trouvé que l'on peut obtenir des pâtes souples ayant une teneur élevée en cire(s) en malaxant la pâte pendant le refroidissement, c'est-à-dire que, pendant au moins une partie du refroidissement, la pâte est malaxée pour créer dans la masse des zones d' écrasement de la pâte. Il faut noter que l'on ne peut pas obtenir une pâte souple convenable par simple agitation avec cisaillement à l'aide d'un agitateur.

Par conséquent, la présente invention a aussi pour objet un procédé de fabrication d'une composition cosmétique sous forme de pâte souple, ayant une teneur élevée en cire(s), telle que définie ci-dessus, dans lequel on prépare un mélange d'au moins une cire et d'au moins un composant pris dans le groupe formé par des constituants gras, des pigments, des charges et des additifs en le portant à une température à laquelle la (ou les) cire(s) du mélange est (sont) fondue(s), puis on le refroidit, caractérisé par le fait que l'on soumet ledit mélange à un malaxage pendant au moins une partie du refroidissement.

Il semble que, dans ces conditions, la cire cristallise sous forme de fins cristaux, ce qui expliquerait que la composition reste sous forme de pâte souple. Cette hypothèse ne doit, néanmoins, aucunement être considérée comme limitative de l'invention.

Pour effectuer le malaxage, on peut, notamment, utiliser deux types d'appareil : un broyeur à cylindres comportant deux cylindres tournant en sens inverse entre lesquels passe la pâte et un mélangeur-extrudeur à vis. On utilise, de préférence, un mélangeur-extrudeur car on obtient une pâte de qualité très constante de façon reproductible. De plus, il est possible, en adaptant la filière de sortie du mélangeur-extrudeur, de conditionner la composition en ligne à la sortie dudit mélangeur-extrudeur.

Selon l'invention, on utilise, de préférence, un mélangeur-extrudeur-cuiseur comportant dans une enveloppe externe munie en sortie d'une filière d'extrusion, un (ou deux) arbre(s) entraîné(s) en rotation de façon que la structure périphérique d'un arbre coopère avec l'enveloppe externe et, le cas échéant, avec la structure périphérique de l'autre arbre, pour assurer le mélange et le malaxage de la pâte et son déplacement dans l'enveloppe vers la filière d'extrusion.

De préférence, l'arbre (ou chacun des arbres) est constitué d'au moins deux manchons successifs, dont la partie intérieure s'adapte sur un axe entraîné en rotation et dont la partie extérieure peut avoir diverses structures périphériques ; parmi les structures classiques, on peut citer, d'une part, un filetage de vis hélicoïdale, dont le pas conduit la matière traitée de l'entrée vers la sortie du mélangeur (désigné plus loin par "DF"), d'autre part, un filetage de vis hélicoïdale à pas inverse du précédent (désigné plus loin par "CF" avec une valeur de pas négative) qui repousse la matière traitée dans le sens allant de la sortie vers l'entrée du mélangeur, un tel filetage comportant des rainures longitudinales pour assurer le passage de la matière vers la sortie du mélangeur, et enfin, un tronçon multilobé comportant sur toute sa longueur des palettes (ou lobes) disposées côte à côte et angulairement décalées les unes par rapport aux autres. Un tronçon bilobé, qui comporte une succession de lobes décalés de 90° les uns par rapport aux autres, est désigné ci-après par "BL". On peut disposer d'un assez grand nombre de manchons à filetage externe pour faire varier le pas, la profondeur et le nombre de filets dans les différentes zones longitudinales successives du mélangeur. De plus, les différentes zones longitudinales du mélangeur peuvent être chauffées par un ou plusieurs fourreaux disposés à l'extérieur de l'enveloppe externe. Le chauffage peut être effectué dans chaque fourreau à l'aide d'au moins une résistance électrique ou d'au moins un échangeur de chaleur.

Selon l'invention, l'arbre (ou chaque arbre) du mélangeur extrudeur-cuiseur utilisé comporte, de préférence, au moins un manchon "DF" formant une vis de transport situé du côté de l'alimentation (ou entrée) du mélangeur, au moins un manchon "CF" (dit "à contrefilet") et/ou un manchon "BL" multilobe ayant une action de malaxage et/ou d'homogénéisation, et au moins un manchon "DF" formant une vis de transport situé à l'extrémité de sortie du mélangeur.

Selon un premier mode de mise en oeuvre, l'invention concerne un procédé du type ci-dessus défini caractérisé par le fait que, dans une première étape, on réalise le mélange de tous les constituants de la composition à une température à laquelle la (ou les) cire(s) est (sont) fondue(s) et, dans une deuxième étape, on introduit le mélange chaud obtenu dans un broyeur à cylindres ou dans un mélangeur-extrudeur à vis.

Selon un autre mode de mise en oeuvre, le procédé selon l'invention est caractérisé par le fait que l'on introduit, en tête d'un mélangeur-extrudeur à vis, un mélange des constituants non pulvérulents de la composition, à une température à laquelle la (ou les) cire(s) est (sont) fondue(s), et on introduit les constituants pulvérulents de la composition dans ledit mélangeur-extrudeur à vis en un ou plusieurs points avant la filière d'extrusion.

On va décrire ci-après, à titre purement illustratif et non limitatif, plusieurs exemples de réalisation selon l'invention.

On opère la préparation des compositions selon l'invention, définies dans les exemples 1 à 3 ci-après, dans un mélangeur extrudeur-cuiseur à deux vis (type "BC 21" de la société "CLEXTRAL"), dont la structure est celle schématisée ci-dessous :

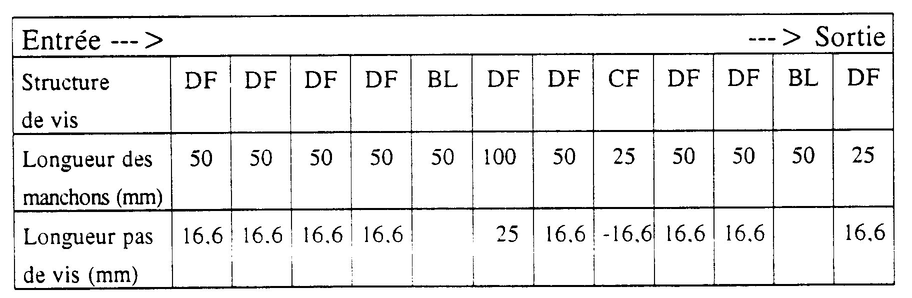

Sur le dessin annexé :
- les figures 1, 3 et 5 représentent en élévation des tronçons de différents types de manchons utilisés sur les arbres du mélangeur mis en oeuvre ;
- les figures 2, 4 et 6 représentent respectivement des coupes transversales selon II-II, IV-IV et VI-VI des figures 1, 3 et 5.

En se référant au dessin, on voit que l'on a désigné par 1 l'enveloppe externe du mélangeur et par 2a, 2b les axes des deux arbres parallèles, qui y sont disposés. Sur les axes 2a, 2b sont enfilés des manchons adjacents, les deux arbres étant équipés des mêmes manchons sur un même tronçon de la longueur pour coopérer mécaniquement entre eux au cours de la rotation.

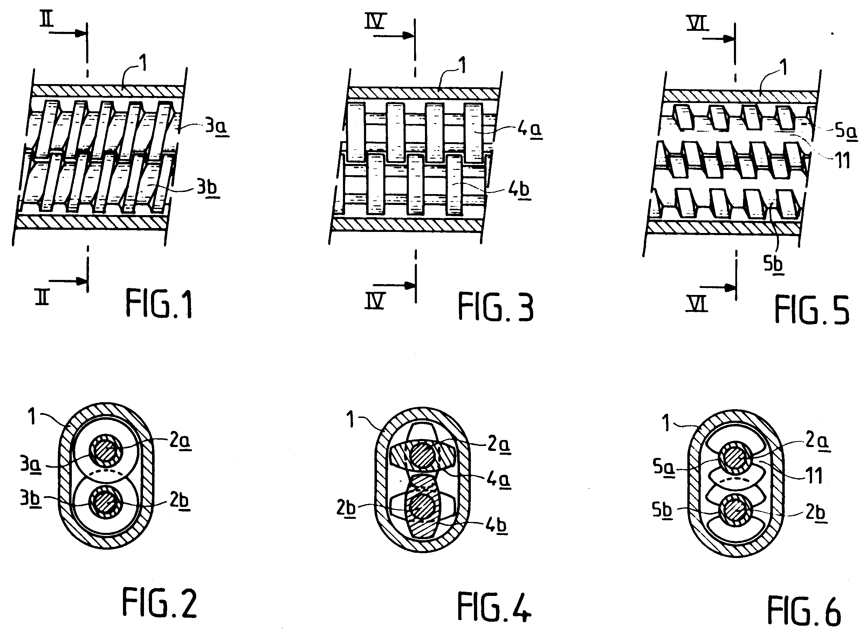

Sur les figures 1 et 2, on a représenté un tronçon où se trouvent des manchons de type "DF" référencés 3a, 3b. Sur les figures 3 et 4, on a représenté un tronçon où se trouvent des manchons 4a, 4b de type bilobé "BL". Sur les figures 5 et 6, on a représenté un tronçon où se trouvent des manchons 5a, 5b de type "CF" avec des rainures longitudinales 11.

Dans le tableau ci-dessus donné :
- - DF: représente un élément de vis à double filet hélicoïdal, tel qu'illustré sur les figures 1 et 2 ;
- - BL: représente un élément bilobé, tel qu'illustré sur les figures 3 et 4; et
- - CF: représente un élément de vis à pas inverse de DF, tel qu'illustré sur les figures 5 et 6, comportant des rainures longitudinales 11.

Les différents éléments ont un diamètre externe de 25 mm, un diamètre intérieur de 14 mm ; la distance entre les axes des deux arbres est de 21 mm.

Les deux arbres tournent à la vitesse de 300 t/min ; les orifices de sortie ont une section totale de 500 mm² ; le débit est de 5 kg/h environ.

Le mélangeur est chauffé à 100°C sur les 300 premiers mm, puis à 30°C sur les 300 mm restants.

### Exemple 1 : Base traitante pour les lèvres

On a préparé une base traitante ayant la formulation suivante (% en poids) :

| *Constituants gras* : | |
|---|---|
| - Huile de vaseline | 22 % |
| - Huile de lanoline | 23,6 % |
| - Lanolate d'isopropyle | 24,2 % |

| *Cires* : | |
|---|---|
| - Cire microcritalline | 15 % |
| - Cire de Carnauba | 15 % |

| *Additifs* : | |
|---|---|
| - Ditertiobutyl hydroxy 4 toluène | 0,2 % |

Cette composition contient 30 % en poids de cires.

On a mélangé les différents constituants à une température de 100°C et on a introduit ce prémélange en tête du mélangeur-extrudeur précédemment défini.

On obtient une pâte souple ayant une viscosité dynamique de 17 Pa.s mesurée à l'aide d'un viscosimètre rotatif CONTRAVES TV équipé d'un mobile "MS-r4" à la fréquence de 60 Hz, qui peut facilement être prélevée à l'aide d'un pinceau pour le traitement dermopharmaceutique des lèvres.

### Exemple 2 : Rouge à lèvres

On a préparé un rouge à lèvres ayant la formulation suivante (% en poids) :

| *Constituants gras* : | |
|---|---|
| - Huile de jojoba | 12 % |
| - Huile de ricin | 7 % |
| - Lanolate d'isopropyle | 20 % |

| *Cires* : | |
|---|---|
| - Cire d'abeilles | 20 % |
| - Cire de polyéthylène | 20 % |

| *Polymères* : | |
|---|---|
| - Polybutène | 8 % |

| *Additif* : | |
|---|---|
| - Butylhydroxytoluène | 0,2 % |

| *Pigments* : | |
|---|---|
| - FD & C Yellow 6 Aluminium lake (CI 15 985) | 1 % |
| - D & C Red 27 (CI 45 410) | 9 % |
| - Dioxyde de titane (CI 77 891) | 2,8 % |

Cette composition contient 40 % en poids de cires.

On a broyé les différents pigments dans les constituants gras (huile de ricin, huile de jojoba et lanolate d'isopropyle). On a rajouté ensuite l'additif et on a chauffé le mélange à 100°C.

On a ensuite introduit les cires dans le mélange préchauffé et on a introduit le tout en tête du mélangeur-extrudeur.

On a obtenu une pâte souple ayant une viscosité dynamique de 24 Pa.s mesurée à l'aide d'un viscosimètre rotatif CONTRAVES TV équipé d'un mobile "MS-r4" à la fréquence de 60 Hz facilement prélevable à l'aide d'un stylo-mousse pour le maquillage des lèvres.

### Exemple 3 : Rouge à lèvres

On a préparé un rouge à lèvres ayant la formulation suivante (% en poids) :

| *Constituants gras* : | |
|---|---|
| - Huile de jojoba | 20 % |
| - Huile de vaseline | 13,8 % |
| - Lanoline | 22 % |

| *Additif* : | |
|---|---|
| - Butylhydroxytoluène | 0,2 % |

| *Charges* : | |
|---|---|
| - Talc | 8 % |
| - Poudre de nylon | 8 % |

| *Cire* : | |
|---|---|
| - Cire microcristalline | 17 % |

| *Pigments* : | |
|---|---|
| - D & C Red 7 calcium lake (CI 15 850:1) | 5,8 % |
| - Oxyde de fer noir (CI 77 499) | 0,2 % |
| - D & C Yellow 6 Aluminium lake (CI 15 985) | 5 % |

Cette composition contient 17 % en poids de cire.

On a préparé un prémélange à 100°C avec les constituants gras, la cire et l'additif et on a introduit ce prémélange par un premier orifice en tête d'extrudeur. On a introduit, en tête d'extrudeur par un second orifice les pigments, et les charges ont été introduites à 100 mm en amont de la filière par un troisième orifice.

On a obtenu une pâte souple ayant une viscosité dynamique de 8 Pa.s mesurée à l'aide d'un viscosimètre rotatif CONTRAVES TV équipé d'un mobile "MS-r4" à la fréquence de 60 Hz facilement prélevable à l'aide d'un stylo-mousse pour le maquillage des lèvres.

## Revendications

1. Composition cosmétique ou dermopharmaceutique sous forme de pâte souple, utilisable pour le maquillage ou le traitement de la peau ou des lèvres, comprenant au moins une cire dans une phase grasse, caractérisée par le fait qu'elle contient de 12 % à 60 % en poids, par rapport au poids total de la composition, d'au moins une cire ayant un point de fusion supérieur à 55°C et qu'elle a une viscosité dynamique à 25°C comprise entre 3 et 30 Pa.s.

2. Composition selon la revendication 1, caractérisée par le fait qu'elle contient de 15 % à 40 % en poids par rapport au poids total de la composition d'au moins une cire.

3. Composition selon l'une des revendications 1 ou 2, caractérisée par le fait que la (les) cire(s) a (ont) un point de fusion compris entre 55 et 110°C et un indice de pénétration à l'aiguille compris entre 3 et 40 telle que mesurée selon la norme française NFT 004.

4. Composition selon l'une des revendications 1 à 3, caractérisée par le fait que la (les) cire(s) est (sont) choisie(s) dans le groupe formé par les cires d'abeille, les cires de Carnauba, de Candellila, d'Ourricury, les cires de fibres de liège, les cires de canne à sucre, les cires du Japon, les cires microcristallines, les cires de lignite, les ozokérites, les cires de polyéthylène et les cires obtenues par synthèse de Fischer et Tropsch.

5. Composition selon l'une des revendications 1 à 4, caractérisée par le fait que la phase grasse contient au moins un constituant gras autre qu'une cire, ledit constituant gras ayant un point de fusion inférieur à 55°C.

6. Composition selon la revendication 5, caractérisée par le fait que le constituant gras est une huile choisie dans le groupe formé par l'huile de paraffine, l'huile de vaseline, les huiles minérales ayant un point d'ébullition compris entre 310 et 410°C ; le perhydrosqualène ; l'huile d'amande douce, l'huile de calophyllum, l'huile de palme, l'huile d'avocat, l'huile de jojoba, l'huile d'olive, l'huile de ricin, les huiles de germes de céréales ; les huiles de silicone ; l'huile de Purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le dicaprylate de propylèneglycol, l'adipate de diisopropyle ; l'alcool oléique, l'alcool linoléique, l'alcool linolénique, l'alcool isostéarylique, l'octyldodécanol ; le lanolate d'isopropyle, le lanolate d'isocétyle ; les acétylglycérides, les octanoates et décanoates de glycol ou de glycérol, le ricinoléate de cétyle, l'oléate d'isopropyle et l'oléate d'isocétyle.

7. Composition selon la revendication 5, caractérisée par le fait que le constituant gras est un corps gras choisi dans le groupe formé par l'huile de ricin hydrogénée, l'huile de palme hydrogénée, le suif hydrogéné, l'huile de coco hydrogénée, le myristate de propylèneglycol, le myristate de myristyle, l'alcool cétylique, les mono-, di-, triglycérides, les sucroglycérides et les lanolines.

8. Composition selon l'une des revendications 5 à 7, caractérisée par le fait que le (les) constituant(s) gras représente(nt) 10 à 90 % en poids de la composition.

9. Composition selon l'une des revendications 1 à 8, caractérisée par le fait qu'elle contient au moins un agent de coloration pulvérulent et/ou au moins une charge minérale ou organique pulvérulente.

10. Composition selon la revendication 9, caractérisée par le fait que l'agent de coloration contient au moins un pigment coloré choisi dans le groupe formé par les oxydes de chrome, les oxydes de fer noir, rouge et jaune, les outremers, le pyrophosphate de manganèse, le bleu ferrique, le dioxyde de titane, une poudre d'argent et une poudre d' aluminium.

11. Composition selon l'une des revendications 9 ou 10, caractérisée par le fait que l'agent de coloration contient au moins un agent nacrant et/ou au moins un colorant organique.

12. Composition selon l'une des revendications 9 à 11, caractérisée par le fait que l'agent de coloration représente de 0,5 à 20 % en poids par rapport au poids total de la composition.

13. Composition selon la revendication 9, caractérisée par le fait que la charge est choisie dans le groupe formé par le talc, les micas, le kaolin, les oxydes de zinc ou de titane, le carbonate de calcium précipité, le carbonate et l'hydrocarbonate de magnésium, la silice, le dioxyde de titane, les billes de verre ou de céramique, les savons métalliques, les poudres de polymères synthétiques non expansées, les poudres de polymères synthétiques expansées et les poudres de matériaux organiques naturels.

14. Composition selon l'une des revendications 9 à 13, caractérisée par le fait que la (les) charge(s) représente(nt) jusqu'à 40 % en poids par rapport au poids total de la composition.

15. Composition selon l'une des revendications 1 à 14, caractérisée par le fait qu'elle contient au moins un adjuvant de formulation et/ou au moins un actif cosmétique et/ou dermopharmaceutique.

16. Composition selon la revendication 15, caractérisée par le fait que l'adjuvant de formulation est un agent antioxydant.

17. Composition selon la revendication 16, caractérisée par le fait que l'agent antioxydant représente moins de 3 % en poids par rapport au poids total de la composition.

18. Composition selon l'une des revendications 15 à 17, caractérisée par le fait que l'actif cosmétique et/ou dermopharmaceutique est choisi dans le groupe formé par les dérivés de vitamine, les acides gras essentiels, les sphingocérils et les filtres solaires liposolubles.

19. Composition selon l'une des revendications 15 à 18, caractérisée par le fait que l'actif cosmétique et/ou dermopharmaceutique représente de 0,05 % à 5 % en poids par rapport au poids total de la composition.

20. Procédé de fabrication d'une composition selon l'une des revendications 1 à 19, dans lequel on prépare un mélange de 12-60% en poids d'au moins une cire ayant un point de fusion supérieur à 55°C et d'au moins un composant pris dans le groupe formé par des constituants gras, des pigments, des charges et des additifs puis on le refroidit, caractérisé par le fait que l'on soumet ledit mélange à un malaxage pendant au moins une partie du refroidissement.

21. Procédé selon la revendication 20, caractérisé par le fait qu'on effectue le malaxage dans un broyeur à cylindres.

22. Procédé selon la revendication 20, caractérisé par le fait qu'on effectue le malaxage dans un mélangeur-extrudeur à vis.

23. Procédé selon la revendication 22, caractérisé par le fait qu'on effectue le malaxage dans un mélangeur extrudeur-cuiseur comportant, dans une enveloppe externe (1) munie en sortie d'une filière d'extrusion, un (ou deux) arbre(s) entraîné(s) en rotation de façon que la structure périphérique d'un arbre coopère avec l'enveloppe externe (1) et, le cas échéant, avec la structure périphérique de l'autre arbre, pour assurer le mélange et le malaxage de la pâte et son déplacement dans l'enveloppe externe (1) vers la filière d'extrusion.

24. Procédé selon la revendication 23, caractérisé par le fait que l'(les) arbre(s) est (sont) constitué(s) d'au moins deux manchons successifs (3a, 3b ; 4a, 4b ; 5a, 5b) dont la partie intérieure s'adapte sur un axe (2a, 2b) entraîné en rotation et la partie extérieure a une structure périphérique différente selon le manchon.

25. Procédé selon l'une des revendications 23 ou 24, caractérisé par le fait que l'arbre (ou les arbres) comporte(nt) au moins un manchon (3a, 3b) formant une vis de transport situé du côté alimentation du mélangeur, au moins un manchon (5a, 5b) à contrefilet et/ou au moins un manchon (4a, 4b) multilobe, et au moins un manchon (3a, 3b) formant une vis de transport situé à l'extrémité de sortie du mélangeur.

26. Procédé selon la revendication 20, caractérisé par le fait que, dans une première étape, on réalise le mélange de tous les constituants de la composition à une température à laquelle la (ou les) cire(s) est (sont) fondue(s) et, dans une deuxième étape, on introduit le mélange chaud obtenu dans un broyeur à cylindres ou dans un mélangeur-extrudeur à vis.

27. Procédé selon l'une des revendications 23 à 25, caractérisé par le fait que l'on introduit, en tête d'un mélangeur-extrudeur à vis, un mélange des constituants non pulvérulents de la composition, à une température à laquelle la (ou les) cire(s) est (sont) fondue(s), et on introduit les constituants pulvérulents de la composition dans ledit mélangeur-extrudeur à vis en un ou plusieurs points avant la filière d'extrusion.

## Claims

1. Cosmetic or dermopharmaceutical composition in the form of a soft paste, which can be used for making up or treating the skin or lips, comprising at least one wax in a fatty phase, characterized in that it contains from 12 % to 60 % by weight, relative to the total weight of the composition, of at least one wax having a melting point above 55°C, and in that it has a dynamic viscosity at 25°C of between 3 and 30 Pa.s.

2. Composition according to Claim 1, characterized in that it contains from 15 % to 40 % by weight, relative to the total weight of the composition, of at least one wax.

3. Composition according to either of Claims 1 and 2, characterized in that the wax(es) has/have a melting point of between 55 and 110°C and a needle penetration value of between 3 and 40 as measured according to French Standard NFT 004.

4. Composition according to one of Claims 1 to 3, characterized in that the wax(es) is/are chosen from the group composed of beeswaxes, carnauba, candelilla and ouricury waxes, cork fibre waxes, sugar-cane waxes, Japan waxes, microcrystalline waxes, lignite waxes, ozokerites, polyethylene waxes and the waxes obtained by Fischer-Tropsch synthesis.

5. Composition according to one of Claims 1 to 4, characterized in that the fatty phase contains at least one fatty constituent other than a wax, said fatty constituent having a melting point below 55°C.

6. Composition according to Claim 5, characterized in that the fatty constituent is an oil chosen from the group composed of paraffin oil, liquid petrolatum, mineral oils having a boiling point of between 310 and 410°C; perhydrosqualene; sweet almond oil, calophyllum oil, palm oil, avocado oil, jojoba oil, olive oil, castor oil, cereal-germ oils; silicone oils, purcellin oil, butyl myristate, isopropyl myristate, cetyl myristate, isopropyl palmitate, butyl stearate, hexadecyl stearate, isopropyl stearate, octyl stearate, isocetyl stearate, decyl oleate, hexyl laurate, propylene glycol dicaprylate, diisopropyl adipate; oleyl alcohol, linoleyl alcohol, linolenyl alcohol, isostearyl alcohol, octyl-dodecanol; isopropyl lanolate, isocetyl lanolate; acetyl-glycerides, glycol or glycerol octanoates and decanoates: cetyl ricinoleate, isopropyl oleate and isocetyl oleate.

7. Composition according to Claim 5, characterized in that the fatty constituent is a fat chosen from the group composed of hydrogenated castor oil, hydrogenated palm oil, hydrogenated tallow, hydrogenated coconut oil, propylene glycol myristate, myristyl myristate, cetyl alcohol, mono-, di- and triglycerides, sucroglycerides and lanolins.

8. Composition according to one of Claims 5 to 7, characterized in that the fatty constituent(s) represent(s) 10 to 90 % by weight of the composition.

9. Composition according to one of Claims 1 to 8, characterized in that it contains at least one pulverulent colouring agent and/or at least one pulverulent inorganic or organic filler.

10. Composition according to Claim 9, characterized in that the colouring agent contains at least one coloured pigment chosen from the group composed of chromium oxides, black, red and yellow iron oxides, ultramarines, manganese pyrophosphate, ferric blue, titanium dioxide, a silver powder and an aluminium powder.

11. Composition according to either of Claims 9 and 10, characterized in that the colouring agent contains at least one pearlescent agent and/or at least one organic dye.

12. Composition according to one of Claims 9 to 11, characterized in that the colouring agent represents from 0.5 to 20 % by weight relative to the total weight of the composition.

13. Composition according to Claim 9, characterized in that the filler is chosen from the group composed of talc, micas, kaolin, zinc or titanium oxides, precipitated calcium carbonate, magnesium carbonate and magnesium hydrogen carbonate, silica, titanium dioxide, glass or ceramic beads, metal soaps, non-expanded powders of synthetic polymers, expanded powders of synthetic polymers and powders of natural organic materials.

14. Composition according to one of Claims 9 to 13, characterized in that the filler(s) represent(s) up to 40 % by weight relative to the total weight of the composition.

15. Composition according to one of Claims 1 to 14, characterized in that it contains at least one formulation adjuvant and/or at least one cosmetic and/or dermopharmaceutical active agent.

16. Composition according to Claim 15, characterized in that the formulation adjuvant is an antioxidant.

17. Composition according to Claim 16, characterized in that the antioxidant represents less than 3 % by weight relative to the total weight of the composition.

18. Composition according to one of Claims 15 to 17, characterized in that the cosmetic and/or dermopharmaceutical active agent is chosen from the group composed of vitamin derivatives, essential fatty acids, sphingo-cerils and sunscreen agents which are fat-soluble.

19. Composition according to one of Claims 15 to 18, characterized in that the cosmetic and/or dermopharmaceutical active agent represents from 0.05 % to 5 % by weight relative to the total weight of the composition.

20. Process for the manufacture of a composition according to one of Claims 1 to 19, in which a mixture of 12 to 60 % by weight of at least one wax having a melting point above 55°C and of at least one component taken from the group composed of fatty constituents, pigments, fillers and additives is prepared and then cooled, characterized in that the said mixture is subjected to a kneading for at least a part of the cooling.

21. Process according to Claim 20, characterized in that the kneading is performed in a roll mill.

22. Process according to Claim 20, characterized in that the kneading is performed in a screw extruder-mixer.

23. Process according to Claim 22, characterized in that the kneading is performed in a cooker-extruder-mixer containing, in an outer barrel (1) equipped on the exit side with an extrusion die, one (or two) shaft(s) driven in rotation so that the peripheral structure of one shaft cooperates with the outer barrel (1) and, where appropriate, with the peripheral structure of the other shaft to effect the mixing and kneading of the paste and its movement along the outer barrel (1) towards the extrusion die.

24. Process according to Claim 23, characterized in that the shaft(s) consist(s) of at least two successive sleeves (3a, 3b; 4a, 4b; 5a, 5b), the inside portion of which fits over a spindle (2a, 2b) driven in rotation, and the outside portion has a different peripheral structure, depending on the sleeve.

25. Process according to either of Claims 23 and 24, characterized in that the shaft (or shafts) possess(es) at least one sleeve (3a, 3b) forming a transport screw situated on the feed side of the mixer, at least one counterflight type sleeve (5a, 5b) and/or at least one multilobe sleeve (4a, 4b), and at least one sleeve (3a, 3b) forming a transport screw situated at the exit end of the mixer.

26. Process according to Claim 20, characterized in that, in a first step, the mixing of all the constituents of the composition is carried out at a temperature at which the wax(es) is/are molten, and, in a second step, the hot mixture obtained is introduced into a roll mill or into a screw extruder-mixer.

27. Process according to one of Claims 23 to 25, characterized in that a mixture of the non-pulverulent constituents of the composition is introduced at the head of a screw extruder-mixer at a temperature at which the wax(es) is/are molten, and the pulverulent constituents of the composition are introduced into the said screw extruder-mixer at one or more points before the extrusion die.

## Patentansprüche

1. Kosmetische oder dermatologische Zusammensetzung in Form einer weichen Paste zum Schminken oder zur Behandlung der Haut oder der Lippen, umfassend wenigstens ein Wachs in einer Fettphase, dadurch gekennzeichnet, dass sie 12 bis 60 Gew.-%, bezogen auf das Gesamtgewicht der Zusammensetzung, wenigstens eines Wachses mit einem Schmelzpunkt von mehr als 55 °C enthält und eine dynamische Viskosität bei 25 °C zwischen 3 und 30 Pa·s aufweist.

2. Zusammensetzung nach Anspruch 1, dadurch gekennzeichnet, dass sie 15 bis 40 Gew.-%, bezogen auf das Gesamtgewicht der Zusammensetzung, wenigstens eines Wachses enthält.

3. Zusammensetzung nach Anspruch 1 oder 2, dadurch gekennzeichnet, dass das (die) Wachs(e) einen Schmelzpunkt zwischen 55 und 110 °C und einen Nadelpenetrationsindex, gemessen nach der französischen Norm NFT 004, zwischen 3 und 40 aufweist (aufweisen).

4. Zusammensetzung nach einem der Ansprüche 1 bis 3, dadurch gekennzeichnet, dass das (die) Wachs(e) ausgewählt ist (sind) unter den Bienenwachsen, Carnaubawachsen, Candelillawachsen, Ouricurywachsen, Korkfaserwachsen, Zuckerrohrwachsen, Japanwachsen, mikrokristallinen Wachsen, Lignitwachsen, Ozokeriten, Polyethylenwachsen und Fischer-Tropsch-Wachsen.

5. Zusammensetzung nach einem der Ansprüche 1 bis 4, dadurch gekennzeichnet, dass die Fettphase wenigstens einen von Wachs verschiedenen Fettbestandteil enthält, welcher einen Schmelzpunkt von weniger als 55 °C aufweist.

6. Zusammensetzung nach Anspruch 5, dadurch gekennzeichnet, dass es sich bei dem Fettbestandteil um ein Öl handelt, das ausgewählt ist unter Paraffinöl, Vaselinöl, Mineralölen mit einem Siedepunkt zwischen 310 und 410 °C, Perhydrosqualen, Süßmandelöl, Calophyllöl, Palmöl, Avocadoöl, Jojobaöl, Olivenöl, Rizinusöl, Getreidekeimölen, Silikonölen, Purcellinöl, Butylmyristat, Isopropylmyristat, Cetylmyristat, Isopropylpalmitat, Butylstearat, Hexadecylstearat, Isopropylstearat, Octylstearat, Isocetylstearat, Decyloleat, Hexyllaurat, Propylenglykoldicraprylat, Diisopropyladipat, Oleylalkohol, Linoleylalkohol, Linolenylalkohol, Isostearylalkohol, Octyldodecanol, Isopropyllanolat, Isocetyllanolat, Acetylglyceriden, Glykol- oder Glyceroloctanoaten und -decanoaten, Cetylrizinoleat, Isopropyloleat und Isocetyloleat.

7. Zusammensetzung nach Anspruch 5, dadurch gekennzeichnet, dass es sich bei dem Fettbestandteil um einen Fettkörper handelt, der ausgewählt ist unter hydriertem Rizinusöl, hydriertem Palmöl, hydriertem Talg, hydriertem Kokosöl, Propylenglykolmyristat, Myristylmyristat, Cetylalkohol, Mono-, Di-, Triglyceriden, Sucroglyceriden und Lanolinen.

8. Zusammensetzung nach einem der Ansprüche 5 bis 7, dadurch gekennzeichnet, dass der (die) Fettbestandteil(e) 10 bis 90 Gew.-% der Zusammensetzung ausmacht (ausmachen).

9. Zusammensetzung nach einem der Ansprüche 1 bis 8, dadurch gekennzeichnet, dass sie wenigstens ein pulverförmiges Farbmittel und/oder wenigstens einen pulverförmigen mineralischen oder organischen Füllstoff enthält.

10. Zusammensetzung nach Anspruch 9, dadurch gekennzeichnet, dass das Farbmittel wenigstens ein Farbpigment enthält, das ausgewählt ist unter Chromoxiden, schwarzen, roten und gelben Eisenoxiden, Ultramarinen, Manganpyrophosphat, Eisenblau, Titandioxid, einem Silberpulver und einem Aluminiumpulver.

11. Zusammensetzung nach einem der Ansprüche 9 oder 10, dadurch gekennzeichnet, dass das Farbmittel wenigstens ein Perlglanzmittel und/oder wenigstens einen organischen Farbstoff enthält.

12. Zusammensetzung nach einem der Ansprüche 9 bis 11, dadurch gekennzeichnet, dass das Farbmittel 0,5 bis 20 Gew.-%, bezogen auf das Gesamtgewicht der Zusammensetzung, ausmacht.

13. Zusammensetzung nach Anspruch 9, dadurch gekennzeichnet, dass der Füllstoff ausgewählt ist unter Talk, Glimmern, Kaolin, Zink- oder Titanoxiden, gefälltem Calciumcarbonat, Magnesiumcarbonat und -hydrogencarbonat, Kieselsäure, Titandioxid, Glas- oder keramischen Kugeln, Metallseifen, Pulvern ungeschäumter synthetischer Polymere, Pulvern geschäumter synthetischer Polymere und Pulvern natürlicher organischer Materialien.

14. Zusammensetzung nach einem der Ansprüche 9 bis 13, dadurch gekennzeichnet, dass der (die) Füllstoff(e) bis zu 40 Gew.-%, bezogen auf das Gesamtgewicht der Zusammensetzung, ausmacht (ausmachen).

15. Zusammensetzung nach einem der Ansprüche 1 bis 14, dadurch gekennzeichnet, dass sie wenigstens einen Formulierungshilfsstoff und/oder wenigstens einen kosmetischen und/oder dermatologischen Wirkstoff enthält.

16. Zusammensetzung nach Anspruch 15, dadurch gekennzeichnet, dass es sich bei dem Formulierungshilfsstoff um ein Antioxidationsmittel handelt.

17. Zusammensetzung nach Anspruch 16, dadurch gekennzeichnet, dass das Antioxidationsmittel wenigstens 3 Gew.-%, bezogen auf das Gesamtgewicht der Zusammensetzung, ausmacht.

18. Zusammensetzung nach einem der Ansprüche 15 bis 17, dadurch gekennzeichnet, dass der kosmetische und/oder dermatologische Wirkstoff ausgewählt ist unter den Vitaminderivaten, essentiellen Fettsäuren, Sphingocerilen und lipidlöslichen Lichtschutzmitteln.

19. Zusammensetzung nach einem der Ansprüche 15 bis 18, dadurch gekennzeichnet, dass der kosmetische und/oder dermatologische Wirkstoff 0,05 bis 5 Gew.-%, bezogen auf das Gesamtgewicht der Zusammensetzung, ausmacht.

20. Verfahren zur Herstellung einer Zusammensetzung nach einem der Ansprüche 1 bis 19, bei dem man ein Gemisch von 12 bis 60 Gew.-% wenigstens eines Wachses mit einem Schmelzpunkt von mehr als 55 °C und wenigstens einem unter der aus Fettbestandteilen, Pigmenten, Füllstoffen und Additiven bestehenden Gruppe ausgewählten Bestandteil herstellt und dieses dann abkühlt, dadurch gekennzeichnet, dass man das Gemisch zumindest während eines Abschnitts der Abkühlung einer Plastifizierung unterwirft.

21. Verfahren nach Anspruch 20, dadurch gekennzeichnet, dass man die Plastifizierung in einer Walzenmühle durchführt.

22. Verfahren nach Anspruch 20, dadurch gekennzeichnet, dass man die Plastifizierung in einem Schneckenmischextruder durchführt.

23. Verfahren nach Anspruch 22, dadurch gekennzeichnet, dass man die Plastifizierung in einem beheizten Mischextruder durchführt, der in einem am Ausgang mit einer Extrusionsdüse versehenen äußeren Gehäuse (1) eine (oder zwei) derartig in Drehung versetzte Welle(n) aufweist, dass das Umfangsprofil einer Welle mit dem äußeren Gehäuse (1) und gegebenenfalls mit dem Umfangsprofil der anderen Welle zusammenwirkt, um das Mischen und die Plastifizierung der Paste und ihre Fortbewegung in dem äußeren Gehäuse (1) zur Extrusionsdüse zu bewirken.

24. Verfahren nach Anspruch 23, dadurch gekennzeichnet, dass die Welle(n) aus mindestens zwei aufeinanderfolgenden Manschetten (3a, 3b; 4a, 4b; 5a, 5b) aufgebaut ist (sind), deren Innenteil an eine in Drehung versetzte Achse (2a, 2b) angepasst ist und deren Außenteil ein je nach Manschette unterschiedliches Umfangsprofil aufweist.

25. Verfahren nach einem der Ansprüche 23 oder 24, dadurch gekennzeichnet, dass die Welle (oder die Wellen) wenigstens eine Manschette (3a, 3b), die eine an der Einspeiseseite des Mischers gelegene Transportschnecke bildet, wenigstens eine gegenläufige Manschette (5a, 5b) und/oder wenigstens eine mehrzackige Manschette (4a, 4b) und mindestens eine Manschette (3a, 3b) aufweist (aufweisen), die eine am Ausgangsende des Mischers gelegene Transportschnecke bildet.

26. Verfahren nach Anspruch 20, dadurch gekennzeichnet, dass man in einer ersten Stufe ein Gemisch aller Bestandteile der Zusammensetzung bei einer Temperatur herstellt, bei der das (oder die) Wachs(e) geschmolzen ist (sind), und in einer zweiten Stufe das erhaltene warme Gemisch in eine Walzenmühle oder einen Schneckenmischextruder einführt.

27. Verfahren nach einem der Ansprüche 23 bis 25, dadurch gekennzeichnet, dass man am Kopf eines Schneckenmischextruders ein Gemisch der nichtpulverförmigen Bestandteile der Zusammensetzung bei einer Temperatur einführt, bei der das (oder die) Wachs(e) geschmolzen ist (sind), und die pulverförmigen Bestandteile der Zusammensetzung an einem oder mehreren Punkten vor der Extrusionsdüse in den Schneckenmischextruder einführt.
